# EUROPEAN PATENT APPLICATION

(11) **EP 1 958 662 A1**
(43) Date of publication of application: **20.08.2008**
(21) Application number: 06834013.2
(22) Date of filing: 05.12.2006
(51) Int. Cl.: A61N 5/06, A61B 17/00, H01S 5/343

(54) **METHOD OF LIGHT THERAPY BY SEMICONDUCTOR LIGHT EMITTING DEVICE AND LIGHT THERAPEUTIC SYSTEM BY SEMICONDUCTOR LIGHT EMITTING DEVICE**

(30) Priority: 05.12.2005 JP 2005350705
(71) Applicant: Meijo University, Nagoya-shi, Aichi 4688-502 (JP); Nagoya City University, Aichi 467-8601 (JP)
(72) Inventor: INADA, Shunko Albano, Yokkaichi-shi, Mie 5121214 (JP); AMANO, Hiroshi, Nagoya-shi, Aichi 4650011 (JP); MORITA, Akimichi, Nagoya-shi, Aichi 4670804 (JP)
(74) Representative: von Hellfeld, Axel
(86) International application number: PCT/JP2006/324258
(87) International publication number: WO 2007/066657

(57) **Abstract**

A system for phototherapy and a method for phototherapy are disclosed. The system redeems the faults of conventional fluorescent tube-type phototherapeutic system. Moreover, the system is small size, lightweight and thus portable, and the system renders a topical irradiation for only a disordered portion of skin possible in combination with a shooting system. A semiconductor ultraviolet ray light-emitting element is prepared, and a given ultraviolet ray is generated from this semiconductor ultraviolet ray light-emitting element and is oscillated, and the disordered site is irradiated in order to treat said disordered site.

## Description

### TECHNICAL FIELD

The invention relates to a method of phototherapy by a semiconductor light-emitting element, and a system of phototherapy with a semiconductor light-emitting element.

### BACKGROUND ART

The phototherapy which belongs to a technical field of this invention has long history, and it is known that Hippocrates used the heliotherapy for prevention of dermatosis around B.C. 460 in ancient times. Neels FINZEN of Denmark used an artificial light first against the treatment using sunrays, i.e., a natural light. Carbon arc lamp was contrived for the first time in 1893, and the remarkable curative effect was confirmed for lupus vulgaris.

In Japan, it is said that the phototherapy with carbon arc lamp is used for the first time in the Tokyo University, depertment of dermatology in 1903. Although every initial apparatus was articles imported, the Japan-made carbon arc lamp is developed by the technical cooperation of the Yoshimasa UTSUNOMIYA and IBIDEN CO., LTD. in 1932 (Showa 7).

As for the artificial light source, including a carbon arc lamp in the phototherapy that had been taken for a long time, a close approximation to the sun light spectrum is used. Moreover, above all, in late years the fluorescent tube which has middle wavelength ultraviolet rays (UV-B) or long wavelength ultraviolet rays (UV-A) in the wavelength area came to be used, and the ultraviolet rays treatment was generalized as a treatment of the disorder of skin. However, it is recognized that it is necessary for treatment to use the rays of a particular wavelength each for various skin diseases as the mechanism of phototherapy is elucidated. The recognition is based on the grounds of that the objective is to minimize a side effect and to maximize an effect.

For example, as one of the hospital which performs phototherapy from the earliest time in the world, the Nagoya City University hospital carries out a treatment of psoriasis by the UV-B wave with a wavelength of 311-313 nm. As for the light source, the fluorescence tube is used, which Philips Corporation of the Netherlands developed. The phototherapy system using the fluorescence tube is characterized in that the system makes a large area irradiation possible and the spectral line width of very narrow ultraviolet light is obtained except that it is limited to only 311-313 nm.

### DISCLOSURE OF INVENTION

### TECHNICAL PROBLEM

However, there are the following problems: (1) the equipment is large-scale and not portable; (2) a big area is required for installation; (3) a normal site is irradiated owing to a large area irradiation; (4) there is a possibility that the medical worker is exposed to irradiation; and (5) the selectivity of wavelength is poor because an available wavelength is restricted by the fluorescence pipe (Today, the ultraviolet light with very narrow spectral line width is only 311-131 nm), and the like. Accordingly, the spread was prohibited. It is an object of this invention to provide a system for phototherapy and a method for phototherapy. The system redeems a fault of the conventional fluorescent tube-type phototherapeutic system. Moreover, the system is small size, lightweight and thus portable, and the system renders a topical irradiation for only a disordered portion of skin possible in combination with a shooting system.

### SOLUTION TO PROBLEM

In order to attain the above-mentioned object, this invention relates to a system of phototherapy with a semiconductor light-emitting element, comprising;
a semiconductor ultraviolet ray light-emitting element for generating a given ultraviolet ray and oscillating the ray,
wherein the system is constructed so as to irradiate a disordered site with the ray to treat the disordered site.

In addition, this invention relates to a method of phototherapy with a semiconductor light-emitting element, comprising the steps of: preparing a semiconductor ultraviolet ray light-emitting element, and generating a given ultraviolet ray from the semiconductor ultraviolet ray light-emitting element and oscillating the ray and irradiating a disordered site with the ray to treat the disordered site.

The inventors have come to develop the semiconductor ultraviolet rays light-emitting element which can make the high-intensity ultraviolet rays generation and emission highly effective in the process of research and development of a semiconductor light-emitting element over many years. This semiconductor ultraviolet rays light-emitting element has the following features.

1. Comparing with the conventional glass tube, it is a) microminiature, b) lightweight, c) point light source, and d) the combination of various wavelengths is possible, e) the intensity variability is easy. Furthermore, f) the emission wavelength range is narrow and it is possible to emit light selectively only in a specific wavelength. 2. The miniaturization of device is easy. 3. The irradiation is possible as a topical method (target type irradiation, and spot delivery). As a result, the irradiation is not performed in a normal site without a lesion, and therefore it is possible to reduce the side effects of irradiation in the normal site. In addition, it is possible to remove the ultraviolet rays exposure to a medical worker (on the part of operator which irradiates).

Therefore, the above-mentioned semiconductor ultraviolet rays light-emitting element has many advantages which resolve the various faults of the conventional fluorescence tube as mentioned above. As a result, the semiconductor ultraviolet rays light-emitting element which has such a feature is used instead of the fluorescence tube mentioned above in the conventional phototherapy, most of the faults based on the fluorescence tube type phototherapy which was mentioned above can be resolved.

Moreover, since an alternative ultraviolet rays wavelength can be used by means of the semiconductor ultraviolet-rays light-emitting element, the additional action and effect can be obtained so that the side effects, such as an erythema reaction, pigmentation, carcinogenicity and the like are reduced to the minimum. Furthermore, since a semiconductor ultraviolet rays light-emitting element is a point light source, the light of uniform intensity can be irradiated to a disordered site. Moreover, since it is small size and lightweight, the position to a disordered site can be determined easily and the distance from the disordered site can be determined easily.

Furthermore, in a mode of the invention, the semiconductor ultraviolet rays light-emitting element can be used to operate the ultraviolet ray on the disordered site. Since it is difficult that the spot of the ultraviolet rays emitted from the semiconductor ultraviolet rays light-emitting element is set always to meet with the size of a disordered site, such a operation can make the irradiation of intended ultraviolet rays possible over the whole disordered site.

Moreover, in another mode of the invention, the semiconductor ultraviolet ray light-emitting element can compose a plurality of semiconductor ultraviolet ray light-emitting elements, wherein these semiconductor ultraviolet ray light-emitting elements are arranged in a shape of array, a portion of the plurality of semiconductor ultraviolet ray light-emitting elements corresponding to the disordered site is turn on, the ultraviolet ray is irradiated over the whole disordered site. As mentioned above, it is difficult that the spot of the ultraviolet rays emitted from the semiconductor ultraviolet rays light-emitting element is set always to meet with the size of a disordered site. Therefore, the plurality of semiconductor ultraviolet-rays light-emitting elements are arranged in a shape of an array, and only the predetermined portion of light-emitting element is turn on, and thereby the intended ultraviolet rays can be irradiated over the whole disordered site.

Furthermore, in still another mode of the invention, a means for imaging a subject to be irradiated with the ultraviolet ray can be composed, wherein the subject to be irradiated with the ultraviolet ray is imaged to obtain a given imaging data and thereafter the disordered site is specified based on this imaging data.

In addition, the method and device of the invention can be used in any disorder, especially preferably in a skin disorder. Specifically, the disorder can be at least one selected from the group consisting of an intractable eczema, a dyshidrotic eczema, a cutaneous T cell lymphoma, an atopic darmatitis, an alopecia areata, a keloid, a cicatrix, an atrophia cutis linear (a strech mark), a scleroderma, a leukoplakia, a psoriasis, a palmoplantar pustulosis, a chronic eczema, "an atopic darmatitis".

As explained above, according to the invention, a system for phototherapy and a method for phototherapy can be provided. The faults of the conventional fluorescence tube-type phototherapy system can be compensated. Moreover, the system is small size, lightweight and thus portable, and the system renders a topical irradiation for only a disordered portion of skin possible in combination with a shooting system.

### BEST MODE FOR CARRYING OUT THE INVENTION

The details and other features and advantages of the invention will be describe in detail based on the best mode as follows.

In the invention, firstly, the predetermined semiconductor ultraviolet rays light-emitting element is prepared. This semiconductor ultraviolet rays light-emitting element can be used solely and can be used to arrange a plurality of the elements in the shape of array.

If the single semiconductor ultraviolet rays light-emitting element is used, it is difficult that in the disordered site the spot of the ultraviolet rays emitted from the semiconductor ultraviolet rays light-emitting element is set always to meet with the size of a disordered site. Therefore, the ultraviolet rays are operated so that the intended ultraviolet rays can be irradiated over the whole disordered site.

In addition, if a plurality of semiconductor ultraviolet rays light-emitting elements are arranged in a shape of array, a portion of the plurality of semiconductor ultraviolet ray light-emitting elements corresponding to the disordered site is turn on, the ultraviolet ray is irradiated over the whole disordered site. As mentioned above, it is difficult that the spot of the ultraviolet rays emitted from the semiconductor ultraviolet rays light- emitting element is set always to meet with the size of a disordered site. Therefore, the plurality of semiconductor ultraviolet rays light-emitting elements is arranged in a shape of an array, and only the predetermined portion of light emitting element is turn on so that the intended ultraviolet rays can be irradiated over the whole disordered site.

Furthermore, if the above-mentioned semiconductor ultraviolet rays light-emitting element is used, the peak wavelength exists within at least one extend of a range of from 350 nm to 390 nm, from 305 nm to 315 nm and from 200 nm to 305 nm. In other words, the above-mentioned semiconductor ultraviolet rays light-emitting element has an emitting wavelength within such a wavelength extend and thereby becoming useful for the treatment of above-mentioned disorders, in particular the skin disorder.

Specifically, if the peak wavelength of the semiconductor ultraviolet rays light-emitting element is within the extend of from 350 nm to 390 nm, the element is effective for the treatment of an intractable eczema, a dyshidrotic eczema, a cutaneous T cell lymphoma, an atopic darmatitis, an alopecia areata, a keloid, a cicatrix, an atrophia cutis linear (a strech mark), a scleroderma, or the like. In addition, if the peak wavelength of the semiconductor ultraviolet rays light-emitting element is within the extend of from 305 nm to 315nm, the element is effective for the treatment of a leukoplakia, a psoriasis, a palmoplantar pustulosis, a chronic eczema, an atopic darmatitis or the like.

In addition, the exposure dose of ultraviolet rays emitted from the semiconductor light-emitting element is not limited to a specific dose as long as the above-mentioned disorders such as a skin disorder can be treated. Preferable dose is 1 mW/cm² or more. Hereby, the above-mentioned skin disorder or the like can be treated effectually.

Moreover, the upper limit on intensity of ultraviolet rays is also not limited in particular. 10 W/cm² or less is preferable. If the ultraviolet rays are irradiated over the above-mentioned value, the side effects, such as an erythema reaction, pigmentation, carcinogenicity and the like may be generated so that the curative effect can not be elicited sufficiently.

Furthermore, as the above-mentioned semiconductor ultraviolet rays light-emitting element, any available element can be used. However, under the present conditions, there is almost no practical implementation of semiconductor ultraviolet rays light-emitting element which can make the high-intensity ultraviolet rays generation and emission highly effective. Therefore, it is preferable to use the semiconductor ultraviolet rays light-emitting element developed by the present inventors as explained below in detail.

### BRIEF DESCRIPTION OF THE DRAWINGS

[Figure 1] Fig. 1 is a schematic view of constitution showing an example of the semiconductor ultraviolet rays light-emitting element to use in the invention.
[Figure 2] Fig. 2 is a schematic view showing an outline of the band structure in the valence band of GaN and AIN which are a group III nitride semiconductor.
[Figure 3] Fig. 3 is a similar schematic view showing an outline of the band structure in the valence band of GaN and AIN which are a group III nitride semiconductor.
[Figure 4] Fig. 4 is a schematic view of constitution showing an example of the semiconductor ultraviolet rays light-emitting element to use in the invention.
[Figure 5] Fig.5 is a graph showing the emission spectrum of the semiconductor ultraviolet rays light-emitting element which used in an embodiment.

(The first semiconductor ultraviolet rays light-emitting element)

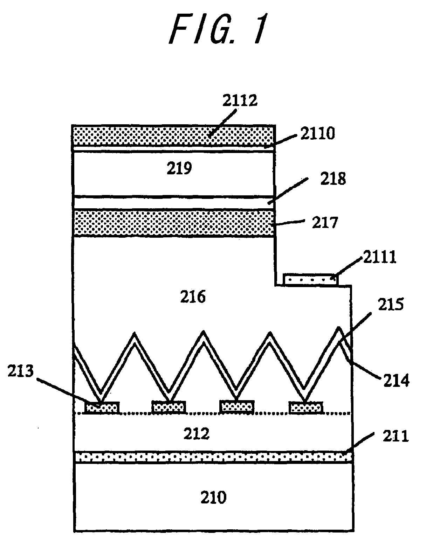

Fig. 1 is a schematic view of constitution showing an example of the semiconductor ultraviolet rays light-emitting element to use in the invention. As shown in Fig. 1, a light-emitting element in this example is an AIGaN based semiconductor light-emitting element. At first, on sapphire substrate 210, AIN layer 211 and AIGaN layer 212 are grown by method of organometallic compounds vapor phase epitaxy, and thereafter SiO₂ mask 213 is formed periodically in the direction of (1-100) on AIGaN layer 212 by an EB vapor deposition device. Subsequently, AIGaN facet layer 214 is formed by method of organic metal vapor phase epitaxy to cover SiO₂ mask 213 completely in order to appear a facet 214 of (11-22).

Subsequently, via AIN layer 215, the planarization is performed by Si-added n-type planarizing layer 216 of Al_{0.50}Ga_{0.50}N showing the n-type conductivity with carrier concentration of 2×10¹⁸cm⁻³, and thereafter a multiquantum well structure active layer 217 of Al_{0.17}Ga_{0.83}N/ Al_{0.25}Ga_{0.75}N, a p-type blocking layer 218 of Al_{0.60}Ga_{0.40}N with carrier density of 8×10¹⁷cm⁻³, a p-type cladding layer 219 of Al_{0.50}Ga_{0.50}N with carrier density of 1×10¹⁸cm⁻³ and a p-type contact layer 2110 of GaN with carrier density of 1×10¹⁸cm⁻³ are laminated in order of precedence, and a n-type electrode 2111 comprising of Ti/Al and a p-type electrode 2112 comprising of Ni/Au are formed and then an AIGaN based semiconductor light-emitting element (a diode) is manufactured.

As for the crystallinity of AIGaN realized by this crystal growth, the dislocation density has a very high quality with 1 x 10⁸cm⁻², and for the p-type blocking layer and p-type cladding layer, a AIGaN is used in AIN molar fraction of 0.6 and 0.5, respectively. Consequently, the p-type blocking layer and the p-type cladding layer will compose of a p-type AIGaN layer of AIN molar fraction of more than 0.15 with a wide gap and a high carrier density of 1×10¹⁸cm⁻³.

The semiconductor ultraviolet rays light-emitting element obtained after this manner shows such an emission property having a peak at 313 nm.

Furthermore, in this example, the p-type blocking layer and the p-type cladding layer have the carrier density of 1×10¹⁸cm⁻³, but the element can generate the ultraviolet rays of sufficient intensity and can emit the light if the requirement of 1×10¹⁶cm⁻³ or more is satisfied. Similarly, the p-type blocking layer and the p-type cladding layer have the AIN molar fraction of 0.6 and 0.5, respectively, but the element can generate the ultraviolet rays of sufficient intensity and can emit the light if the molar fraction is 0.15 or more.

In addition, the p-type blocking layer and the p-type cladding layer have preferably a half bandwidth of 800 seconds or less on X-ray rocking curve of (0002) diffraction, and have preferably a half bandwidth of 1000 seconds or less on X-ray rocking curve of (10-10) diffraction. Hereby, the crystal quality of these layers is improved significantly and thereby the intended high-efficiency ultraviolet rays can be generated and can be emitted.

Moreover, in this example, the p-type AIGaN blocking layer 218 having AlN molar fraction of 0.6 with carrier density of 8×10¹⁷cm⁻³ and the p-type AIGaN cladding layer 219 having AIN molar fraction of 0.5 with carrier density of 1×10¹⁸cm⁻³ are used, but these property values can be properly changed within the scope of the invention in the AIGaN based semiconductor light-emitting element shown in Fig. 1 according to the desired emission wavelength.

Furthermore, as mentioned above, the p-type blocking layer and the p-type cladding layer is composed of the p-type AIGaN layer having the carrier density of more than 5×10¹⁷cm⁻³ and the AIN molar fraction of more than 0.3. Such a p-type AIGaN layer shows the following properties.

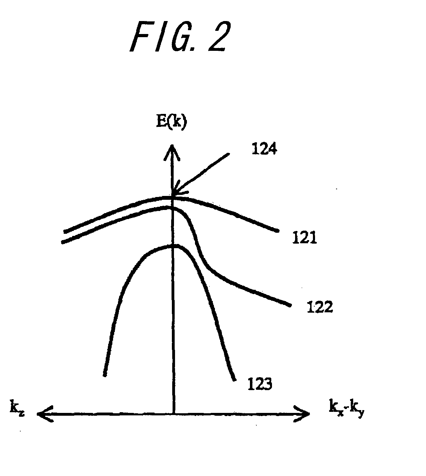

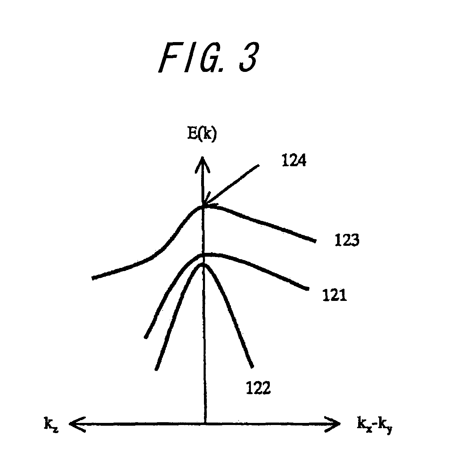

The Figs. 2 and 3 are a schematic view showing an outline of the band structure in the valence band of GaN and AIN which are a group III nitride semiconductor. The band structure in the group III nitride semiconductor is divided to three of a heavy hole (HH) 121, a light hole (LH) 122, and a crystal field splitting hole (CH) 123. In addition, in GaN and AIN, a band of the top at Γ point 124 is HH and CH, respectively and it is a characteristic that these differ each other.

In AIGaN, when an AIN molar fraction is low, the HH and LH are higher than the CH, but as the AIN molar fraction is increased, the CH rises relatively compared with HH and LH, and then these three bands are almost piled up at AIN molar fraction of around 0.40. In addition, if the AIN molar fraction is increased further, the CH is higher than the HH and LH. Based on such properties, if the AIN molar fraction is of from 0 to 0.3, the increase of state density enables the carrier density to decrease, but if the AIN molar fraction is of more than 0.3, the carrier concentration is increased and the maximum value is taken around 0.4, and thereby the AIN becomes also p-type conductive. Therefore, mainly, originating in this kind of p-type AlGaN, the semiconductor ultraviolet ray light-emitting element of this example can make the high-intensity ultraviolet rays emission highly effective.

(The second semiconductor ultraviolet rays light-emitting element)

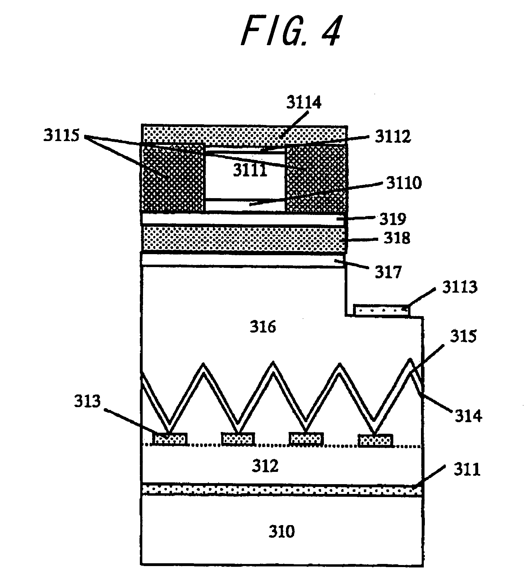

Fig. 4 is a schematic view of constitution showing an example of the semiconductor ultraviolet rays light-emitting element to use in the invention. As shown in Fig. 4, a light-emitting element in this example is also an AIGaN based semiconductor light-emitting element. At first, on sapphire substrate 310, AIN layer 311 and AIGaN layer 312 are grown by method of organometallic compounds vapor phase epitaxy, and then SiO₂ mask 313 is formed periodically in the direction of [1-100] on AIGaN layer 312 by an EB vapor deposition device. Subsequently, AIGaN facet layer 314 is formed by method of organic metal vapor phase epitaxy to appear a AIGaN facet 314 of (11-22) in order to cover SiO₂ mask completely.

Thereafter, via AIN layer 315, the planarization is performed by Si-added n-type planarizing layer 316 of Al_{0.50}Ga_{0.50}N showing the n-type conductivity with carrier concentration of 2×10¹⁸cm⁻³, and then a guide layer 317 of undoped Al_{0.38}Ga_{0.62}N, a multiquantum well structure active layer 318 of Al_{0.17}Ga_{0.83}N/ Al_{0.25}Ga_{0.75}N, a guide layer 319 of undoped Al_{0.38}Ga_{0.62}N, a p-type blocking layer 3110 of Al_{0.60}Ga_{0.40}N with carrier density of 8×10¹⁷cm⁻³, a p-type cladding layer 3111 of Al_{0.50}Ga_{0.50}N with carrier density of 1×10¹⁸cm⁻³ and a p-type contact layer 3112 of GaN with carrier density of 1×10¹⁸cm⁻³ are laminated. Subsequently, a n-electrode 3113 comprising of Ti/Al, a p-electrode 3114 comprising of Ni/Au, an electric current stricture layer comprising of SiO₂ and the like are formed. Consequently, the illustrated AlGaN based semiconductor light-emitting element started functioning as a laser diode of ridge type.

As for the crystallinity of AIGaN realized by this crystal growth, the dislocation density has a very high quality with 1 x 10⁸cm⁻², and for the p-type blocking layer and p-type cladding layer, a AIGaN is used in AIN molar fraction of 0.6 and 0.5, respectively, a wide gap with AIN molar fraction of more than 0.3 and a high carrier density of 8 X 10¹⁷cm⁻³ and 1 × 10¹⁸cm⁻³ can be realized.

The semiconductor ultraviolet rays light-emitting element obtained after this manner shows such an emission property having a peak at 313 nm.

Furthermore, in this example, the p-type blocking layer and the p-type cladding layer have the carrier density of 1×10¹⁸ cm⁻³, but the element can generate the ultraviolet rays of sufficient intensity and can emit the light if the requirement of 1×10¹⁶cm⁻³ or more is satisfied. Similarly, the p-type blocking layer and the p-type cladding layer have the AIN molar fraction of 0.6 and 0.5, respectively, but the element can generate the ultraviolet rays of sufficient intensity and can emit the light if the molar fraction is 0.15 or more.

In addition, the p-type blocking layer and the p-type cladding layer have preferably a half bandwidth of 800 seconds or less on X-ray rocking curve of (0002) diffraction, and have preferably a half bandwidth of 1000 seconds or less on X-ray rocking curve of (10-10) diffraction. Hereby, the crystal quality of these layers is improved significantly and thereby the intended high-efficiency ultraviolet rays can be generated and can be emitted.

Moreover, in this example, the p-type AIGaN blocking layer 218 having AIN molar fraction of 0.6 with carrier density of 8×10¹⁷cm⁻³ and the p-type AIGaN cladding layer 219 having AIN molar fraction of 0.5 with carrier density of 1×10¹⁸cm⁻³ are used, but these property values can be properly changed within the scope of the invention in the AIGaN based semiconductor light-emitting element shown in Fig. 4 according to the desired emission wavelength.

Furthermore, as for the p-type AIGaN layer used in this example, the properties shown in Figs. 2 and 3 are shown.

### EXAMPLES

First, in order to verify that the semiconductor ultraviolet ray light-emitting element is useful for phototherapy, the comparison was performed as to tumor cell death necessary for phototherapy. In the comparison, the conventional broadband UVA irradiation device of partial body UVA1 (340-400nm) irradiation Sellamed system and the light emitting diode which was developed at this time comprising the group III nitride semiconductor with a peak wavelength of 365 nm shown in Fig. 1 are used. The irradiation is performed in the same energy and the size of LED is 0.5mm×0.5mm.

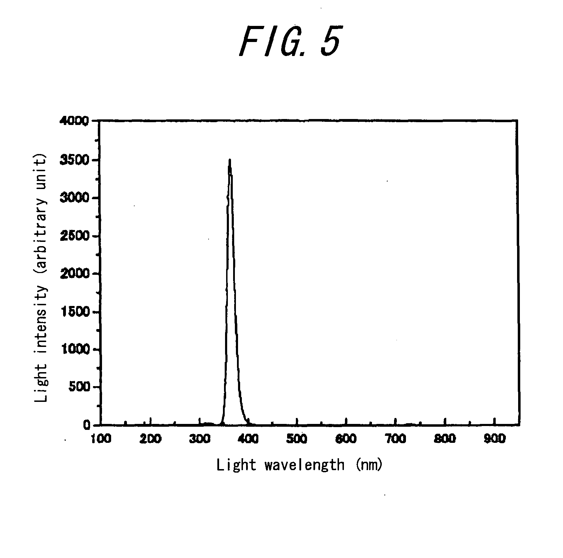

Fig. 5 shows the emission spectrum of the ultraviolet rays LED which used at this time. In Tables 1 and 2, the irradiation intensity of 80 mW/cm², the changes of exposure dose of 10, 20, 30 J/cm², and the ratio of apoptosis caused in tumor cells (Table 1), and the ratio of necrosis (Table 2) by ultraviolet rays irradiation with two light source devices are summarized.

**[Table 1]**

| | UVA1 Ratio of Apoptosis [%] | LED Ratio of Apoptosis [%] | UVA Average | LED Average |
|---|---|---|---|---|
| Control | 19.7 | 19.7 | 19.86 | 19.86 |
| | 20 | 20 | | |
| | 19.87 | 19.87 | | |
| (10)J/cm² | 22.11 | 23.97 | 22.89 | 21.68 |
| | 24.17 | 20.32 | | |
| | 22.4 | 20.74 | | |
| (20)J/cm² | 29.41 | 28.99 | 30.11 | 27.28 |
| | 26.64 | 22.75 | | |
| | 34.29 | 30.09 | | |
| (30)J/cm² | 49.36 | 37.54 | 52.82 | 43.59 |
| | 56.83 | 47.16 | | |
| | 52.28 | 46.06 | | |

**[Table 2]**

| | UVA1 Ratio of Necrosis [%] | LED Ratio of Necrosis [%] | UVA Average | LED Average |
|---|---|---|---|---|
| Control | 20.98 | 20.98 | 20.72 | 20.71 |
| | 19.51 | 19.51 | | |
| | 21.68 | 21.63 | | |
| (10)J/cm² | 24 | 19.68 | 22.08 | 18.47 |
| | 23.95 | 17.52 | | |
| | 18.28 | 18.22 | | |
| (20)J/cm² | 25.57 | 25.74 | 25.90 | 24.72 |
| | 22.35 | 20.77 | | |
| | 29.77 | 27.65 | | |
| (30)J/cm² | 38.28 | 34.61 | 41.87 | 38.37 |
| | 45.61 | 40.35 | | |
| | 41.72 | 40.15 | | |

As is clear from these Tables, LED irradiation device which was developed at this time showed the effect equivalent to the conventional lamp-type broadband UVA irradiation device.

Next, the LEDs of 0.5mm X 0.5mm/one chip were arranged to form the array shape of 20 chips x20 chips, total 400 chips, the area of 20 cm x20 cm. The portion of LEDs was partially lighted, and the above-mentioned irradiation was performed, upon which the apoptosis and necrosis were observed in only the lightning portion of LEDs.

Based on this result, for a patient suffer from an atopic dermatitis, (1) the dermatitis-bearing site was photographed by a digital camera, (2) the obtained photography data was analyzed to prepare a dermatitis map data, (3) a prototype LED a array device was lighted against the site of dermatitis to continue such a treatment of irradiation for 125 seconds, upon which a curative effect was observed.

If energy was the same, it was confirmed that an approximately equal effect was obtained by using LEDs with peak wavelength between from 350 nm to 390nm in this experimentation.

Besides, the effects for various dermatitides were observed in the wavelength regions of from 305 nm to 315 nm, from 315 nm to 350 nm, from 200 nm to 305 nm, respectively. In addition, for various skin diseases, the curative effects were partially observed in a combination of UV-LED and visible LED, and in a combination of UV-LED and infrared rays LED, or a combination of UV-LED, visible LED and infrared rays LED.

As above, the invention has been described in detail on the basis of the forms of the embodiment while listing the concrete examples, but it should be construed that the invention is not limited to the above-mentioned contents and that every variation or change are possible insofar as the scope of invention does not deviate.

### INDUSTRAIL APPLICABILITY

As mentioned above, according to the invention, a system for phototherapy and a method for phototherapy can be provided. The system redeems the faults of conventional fluorescent tube-type phototherapeutic system. Moreover, the system is small size, lightweight and thus portable, and the system renders a topical irradiation for only a disordered portion of skin possible in combination with a shooting system.

## Claims

1. A method of phototherapy with a semiconductor light-emitting element, comprising the steps of:
preparing a semiconductor ultraviolet ray light-emitting element, and
generating a given ultraviolet ray from the semiconductor ultraviolet ray light-emitting element to oscillate the ray in order to irradiate a disordered site with the ray to treat the disordered site.

2. A method of phototherapy with a semiconductor light-emitting element according to claim 1, wherein the semiconductor ultraviolet ray light-emitting element is used to operate the ultraviolet ray on the disordered site.

3. A method of phototherapy with a semiconductor light-emitting element according to claim 1, wherein the semiconductor ultraviolet ray light-emitting element composes a plurality of semiconductor ultraviolet ray light-emitting elements, these semiconductor ultraviolet ray light-emitting elements are arranged in a shape of array, a portion of the plurality of semiconductor ultraviolet ray light-emitting elements corresponding to the disordered site is turn on, the ultraviolet ray is irradiated over the whole disordered site.

4. A method of phototherapy with a semiconductor light-emitting element according to any one of claims 1 to 3, wherein the semiconductor ultraviolet ray light-emitting element has a peak wavelength within at least one extend of a range of from 350 nm to 390 nm, from 305 nm to 315 nm and from 200 nm to 305 nm.

5. A method of phototherapy with a semiconductor light-emitting element according to any one of claims 1 to 4, wherein an ultraviolet ray exposure dose with the semiconductor ultraviolet ray light-emitting element is 1 mW/cm² or more.

6. A method of phototherapy with a semiconductor light-emitting element according to any one of claims 1 to 5, wherein the ultraviolet ray exposure dose with the semiconductor ultraviolet ray light-emitting element is 10 mW/cm² or less.

7. A method of phototherapy with a semiconductor light-emitting element according to any one of claims 1 to 6, wherein the disordered site is a disordered site of skin.

8. A method of phototherapy with a semiconductor light-emitting element according to claim 7, wherein the disordered site is at least one site selected from the group consisting of an intractable eczema, a dyshidrotic eczema, a cutaneous T cell lymphoma, an atopic darmatitis, an alopecia areata, a keloid, a cicatrix, an atrophia cutis linear (a strech mark), a scleroderma, a leukoplakia, a psoriasis, a palmoplantar pustulosis, a chronic eczema, "an atopic darmatitis".

9. A method of phototherapy with a semiconductor light-emitting element according to any one of claims 1 to 8, wherein a subject to be irradiated with the ultraviolet ray is imaged to obtain a given imaging data and thereafter the disordered site is specified based on this imaging data.

10. A method of phototherapy with a semiconductor light-emitting element according to any one of claims 1 to 9, wherein the semiconductor ultraviolet ray light-emitting element is an AIGaN-based semiconductor light-emitting element having a carrier density of more than 5×10¹⁷ cm⁻³, and the element comprises a p-type AIGaN layer specified by an AIN molar fraction of more than 0.15 as at least one of a p-type AIGaN cladding layer and a p-type AIGaN blocking layer.

11. A method of phototherapy with a semiconductor light-emitting element according to claim 10, wherein the semiconductor ultraviolet ray light-emitting element comprises an AIN layer, an AIGaN layer, an AIN layer formed on an AlGaN(11-22) facet, a n-type AIGaN planarizing layer, an AIGaN active layer, the p-type AIGaN blocking layer, and the p-type AIGaN cladding layer in order of precedence on a given substrate.

12. A method of phototherapy with a semiconductor light-emitting element according to claim 10, wherein the semiconductor ultraviolet ray light-emitting element comprises an AIN layer, an AlGaN layer, an AIN layer formed on an AIGaN (11-22) facet, a n-type AIGaN planarizing layer, a first AIGaN guide layer, an AIGaN active layer, a second AIGaN guide layer, the p-type AIGaN blocking layer, and the p-type AIGaN cladding layer in order of precedence on a given substrate.

13. A method of phototherapy with a semiconductor light-emitting element according to claim 12, wherein the semiconductor ultraviolet ray light-emitting element has an electric current stricture formed adjacent to the p-type AIGaN blocking layer and the p-type AIGaN cladding layer, and the element presents a ridge type.

14. A method of phototherapy with a semiconductor light-emitting element according to any one of claims 10 to 13, wherein the p-type AIGaN layer has a half bandwidth of 800 seconds or less on X-ray rocking curve of (0002) diffraction.

15. A method of phototherapy with a semiconductor light-emitting element according to any one of claims 10 to 14, wherein the p-type AIGaN layer has a half bandwidth of 1000 seconds or less on X-ray rocking curve of (10-10) diffraction.

16. A method of phototherapy with a semiconductor light-emitting element according to any one of claims 10 to 15, wherein the p-type AIGaN layer has a dislocation density of 5×10⁹ cm⁻² or less.

17. A method of phototherapy with a semiconductor light-emitting element according to any one of claims 10 to 16, wherein the p-type AIGaN layer satisfies a relation of CH≥HH, LH, wherein HH is a band zone energy of heavy hole, LH is a band zone energy of light hole, and CH is a band zone energy of crystal field splitting hole.

18. A method of phototherapy with semiconductor light-emitting element according to any one of claims 1 to 17, wherein the p-type AIGaN layer has a carrier density of more than 1×10⁸ cm⁻³ or more.

19. A method of phototherapy with a semiconductor light-emitting element according to any one of claims 1 to 18, wherein a subject having the disordered site to be treated with the ultraviolet ray is a animal other than human being.

20. A system of phototherapy with a semiconductor light-emitting element, comprising:
a semiconductor ultraviolet ray light-emitting element for generating a given ultraviolet ray and oscillating the ray,
wherein the system is constructed so as to irradiate a disordered site with the ray to treat the disordered site.

21. A system of phototherapy with a semiconductor light-emitting element according to claim 20, wherein the semiconductor ultraviolet ray light-emitting element is used to operate the ultraviolet ray on the disordered site.

22. A system of phototherapy with a semiconductor light-emitting element according to claim 20, wherein the semiconductor ultraviolet ray light-emitting element composes a plurality of semiconductor ultraviolet ray light-emitting elements, these semiconductor ultraviolet ray light-emitting elements are arranged in a shape of array, a portion of the plurality of semiconductor ultraviolet ray light-emitting elements corresponding to the disordered site is turn on, the ultraviolet ray is irradiated over the whole disordered site.

23. A system of phototherapy with a semiconductor light-emitting element according to any one of claims 20 to 22, wherein the semiconductor ultraviolet ray light-emitting element has a peak wavelength within at least one extend of a range of from 350 nm to 390 nm, from 305 nm to 315 nm and from 200 nm to 305 nm.

24. A system of phototherapy with a semiconductor light-emitting element according to any one of claims 20 to 23, wherein an ultraviolet ray exposure dose with the semiconductor ultraviolet ray light-emitting element is 1 mW/cm² or more.

25. A system of phototherapy with a semiconductor light-emitting element according to any one of claims 20 to 24, wherein the ultraviolet ray exposure dose with the semiconductor ultraviolet ray light-emitting element is 10 mW/cm² or less.

26. A system of phototherapy with a semiconductor light-emitting element according to any one of claims 20 to 25, wherein the disordered site is a disordered site of skin.

27. A system of phototherapy with a semiconductor light-emitting element according to claim 26, wherein the disordered site is at least one site selected from the group consisting of an intractable eczema, a dyshidrotic eczema, a cutaneous T cell lymphoma, an atopic darmatitis, an alopecia areata, a keloid, a cicatrix, an atrophia cutis linear (a strech mark), a scleroderma, a leukoplakia, a psoriasis, a palmoplantar pustulosis, a chronic eczema, "an atopic darmatitis".

28. A system of phototherapy with a semiconductor light-emitting element according to any one of claims 20 to 27, comprising a means for imaging a subject to be irradiated with the ultraviolet ray, wherein the subject to be irradiated with the ultraviolet ray is imaged to obtain a given imaging data and thereafter the disordered site is specified based on this imaging data.

29. A system of phototherapy with a semiconductor light-emitting element according to any one of claims 20 to 28, wherein the semiconductor ultraviolet ray light-emitting element is an AIGaN-based semiconductor light-emitting element having a carrier density of more than 5×10¹⁷ cm⁻³, and the element comprises a p-type AIGaN layer specified by an AIN molar fraction of more than 0.15 as at least one of a p-type AIGaN cladding layer and a p-type AIGaN blocking layer.

30. A system of phototherapy with a semiconductor light-emitting element according to claim 29, wherein the semiconductor ultraviolet ray light-emitting element comprises an AIN layer, an AIGaN layer, an AIN layer formed on an AIGaN (11-22) facet, a n-type AIGaN planarizing layer, an AIGaN active layer, the p-type AIGaN blocking layer, and the p-type AIGaN cladding layer in order of precedence on a given substrate.

31. A method of phototherapy with a semiconductor light-emitting element according to claim 29, wherein the semiconductor ultraviolet ray light-emitting element comprises an AIN layer, an AIGaN layer, an AIN layer formed on an AIGaN (11-22) facet, a n-type AIGaN planarizing layer, a first AIGaN guide layer, an AIGaN active layer, a second AIGaN guide layer, the p-type AIGaN blocking layer, and the p-type AIGaN cladding layer in order of precedence on a given substrate.

32. A system of phototherapy with a semiconductor light-emitting element according to claim 31, wherein the semiconductor ultraviolet ray light-emitting element has an electric current stricture layer formed adjacent to the p-type AIGaN blocking layer and the p-type AIGaN cladding layer, and the element presents a ridge type.

33. A system of phototherapy with a semiconductor light-emitting element according to any one of claims 29 to 32, wherein the p-type AIGaN layer has a half bandwidth of 800 seconds or less on X-ray rocking curve of (0002) diffraction.

34. A system of phototherapy with a semiconductor light-emitting element according to any one of claims 29 to 33, wherein the p-type AIGaN layer has a half bandwidth of 1000 seconds or less on X-ray rocking curve of (10-10) diffraction.

35. A system of phototherapy with a semiconductor light-emitting element according to any one of claims 29 to 34, wherein the p-type AIGaN layer has a dislocation density of 5×10⁹ cm⁻² or less.

36. A system of phototherapy with a semiconductor light-emitting element according to any one of claims 29 to 35, wherein the p-type AIGaN layer satisfies a relation of CH≥HH, LH, wherein HH is a band zone energy of heavy hole, LH is a band zone energy of light hole, and CH is a band zone energy of crystal field splitting hole.

37. A system of phototherapy with a semiconductor light-emitting element according to any one of claims 29 to 36, wherein the p-type AIGaN layer has a carrier density of 1×10⁸ cm⁻³ or more.

38. A system of phototherapy with a semiconductor light-emitting element according to any one of claims 20 to 37, wherein a subject having the disordered site to be treated with the ultraviolet ray is a animal other than human being.
